# EUROPEAN PATENT APPLICATION

(11) **EP 4 579 237 A2**
(43) Date of publication of application: **02.07.2025**
(21) Application number: 25165353.1
(22) Date of filing: 13.03.2018
(51) Int. Cl.: G01N 33/50

(54) **DEVICE, METHOD, AND SYSTEM FOR IDENTIFYING ORGANISMS AND DETERMINING THEIR SENSITIVITY TO TOXIC SUBSTANCES USING THE CHANGES IN THE CONCENTRATIONS OF METABOLITES PRESENT IN GROWTH MEDIUM**

(30) Priority: 13.03.2017 US 201762470756 P
(62) Divisional of application: 18767935.2
(71) Applicant: Lewis, Ian Andrew, Calgary, Alberta T2N 1N4 (CA)
(72) Inventor: Lewis, Ian Andrew, Calgary, Alberta T2N 1N4 (CA)
(74) Representative: Weickmann & Weickmann PartmbB

(57) **Abstract**

Devices, methods and systems are for identifying the cell type of an unknown microorganism. The device includes: an apparatus for culturing unknown organism(s), a diagnostic data acquisition tool and a computer program. The method includes: incubation of the sample with a growth medium (with or without toxins), and an analysis of the metabolites detected in the sample. The computer system compares the results collected from the device to reference metabolite profiles.

## Description

### Field

This invention relates to devices, methods, and systems for detecting consumed and produced metabolites to classify organisms and to measure organism and cell sensitivity to toxic substances.

### Background

Timely identification of cells is useful in many applications. For example, rapid microorganism identification is of great value when considering food safety, genetic engineering research recombinant verification and disease treatment.

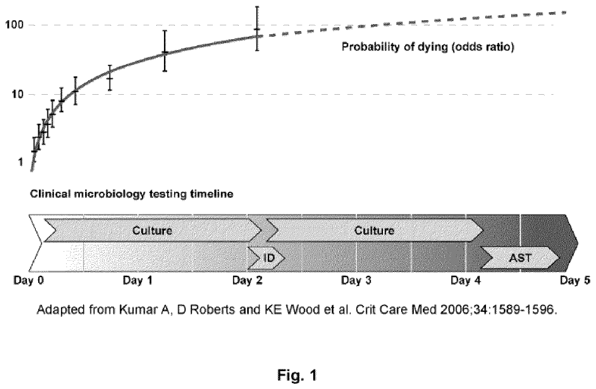

Considering disease treatment, specifically blood borne infection, for example, the length of time between the onset of symptoms and the initiation of effective antibiotic therapy for patients is a major contributor to the morbidity and the mortality from infections. In the case of blood stream infections, survival rates decrease from 80% to 72% over the first 6 hours and continue to decrease hour-by-hour as the infection progresses (Fig. 1).

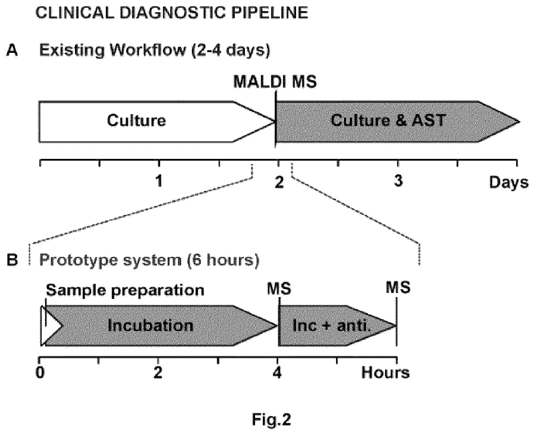

In current practices, such as sample analysis by chemical tests or spectrometric methods such as by matrix assisted laser ionization desorption mass spectrometry (MALDI-MS), it takes 2-4 days to identify an unknown organism and to determine its level of drug sensitivity considering culture time and analysis. Most of the clinical diagnostic timeline for the current practice is spent waiting for microbial cultures to grow (Fig. 2).

### Summary of the Invention

Devices, methods and systems have been invented for identifying living cells such as microorganisms. The devices, methods and systems may also be useful for determining the sensitivity of microorganisms, or other cells, to toxic substances using changes in the concentration of consumed and/or produced metabolites present in a growth medium. The present invention may complete identification and toxin sensitivity testing in approximately half the time compared to the current practice.

In this application, the term "metabolite" is used to mean any substance used in or produced from cellular metabolism. Thus, metabolite includes both nutrients consumed and waste produced by a living cell.

Also in this application, the terms cell, organism, microorganism, microbe, pathogen and bacteria are used interchangeably. These terms refer to one or more microscopically small organisms which may include any of bacteria, fungi, protozoa or other living, isolated cells such as cell suspensions (i.e. excised cells, tissue culture, etc). Note, therefore that the invention can be used for identification of living cells that metabolize in culture such as, for example, bacteria, fungi, protozoa or isolated cells from excised tissue or tissue culture and these cells are collectively often referred to herein as cells or microorganisms. To be clear, the invention is useful for the analysis of living cells, such as: identification of infection causing cells and their response to toxins, identification of food contaminating cells or cancer cells, for example for response to toxins such as chemotherapies.

In this application, "toxin" is any substance that modulates the metabolic activity of a cell. This may a substance that kills a cell as well as a substance that impairs or stimulates a cell function, such as any one or more of metabolic pathways, are ceased or changed to result in a detectable change in metabolic consumption or output.

In accordance with a broad aspect of the present invention, there is provided a method for identifying a cell type of a cell in a sample, comprising: incubating the sample in a growth medium; after incubation, analyzing the incubated growth medium by chemical analysis to determine a level of a metabolite in the incubated growth medium; and identifying the cell type of the cell by comparison of the level of the metabolite with reference metabolite profiles and matching the level of metabolite with a reference metabolite profile indicative of the cell type.

In accordance with another broad aspect of the present invention, there is provided a computer system for identifying a cell type from a clinical sample, the computer system configured to carry out a method comprising: receiving a spectrometric signal indicative of a level of a metabolite in the clinical sample; and comparing the level of metabolite with a database of reference metabolite profiles; matching the level of metabolite with a reference metabolite profile indicative of the cell type; and outputting the cell type for a user.

In accordance with another broad aspect of the present invention, there is provided a device for identifying a cell type of a cell in a sample, the device comprising: a) an analytical data acquisition tool configured for (i) receiving an amount of growth medium that has been incubated with the sample, (ii) conducting a chemical analysis of the growth medium to generate a metabolic profile of the growth medium, and (iii) outputting the metabolic profile; and, b) a processor configured to (i) receive the metabolic profile; (ii) compare the metabolic profile with reference metabolite profiles, to identify the cell type; and (iii) generate an output of the cell type.

It is to be understood that other aspects of the present invention will become readily apparent to those skilled in the art from the following detailed description, wherein various embodiments of the invention are shown and described by way of example. As will be realized, the invention is capable for other and different embodiments and several details of its design and implementation are capable of modification in various other respects, all captured by the present claims. Accordingly, the detailed description and examples are to be regarded as illustrative in nature and not as restrictive.

### Description of the Figures

For a better appreciation of the invention, the following Figures are appended:
**Fig 1****.** Shows microbiology testing timeline versus the probability of patient death due to infection. Survival data are shown between the onset of symptoms and administration of antibiotics. Resistance refers to antibiotic susceptibility testing time and ID refers to microbial identification by MALDI-MS.
**Fig. 2**. (A) Shows clinical workflow for current health care practice for identification of an unknown organism and its toxin sensitivity. The first 1-2 days of culturing are spent waiting for bacteria to grow to detectable densities. MALDI-MS analyses are then completed to identify the unknown organism. An aliquot of the culture is inoculated into new cultures and antibiotic susceptibility testing ("AST") is completed by growing the unknown organisms in several antibiotics over a range of drug doses. (B) Shows a possible timeline for one embodiment of the present invention. The unknown organisms are combined with a nutrient-containing growth medium and incubated for four hours. Metabolite analyses are conducted using an MS instrument. Antibiotic susceptibility testing is completed in the second stage incubation ("Inc + anti"). The unknown organisms identified in the first stage are combined with a controlled dose of antibiotics in fresh growth medium and incubated. Metabolites present in the growth medium are assessed by MS to determine the effective antibiotic dose.

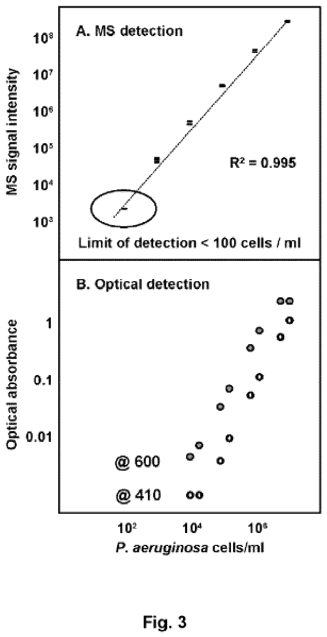

**Fig. 3****.** Shows MS-based and optical-based limits of detection.

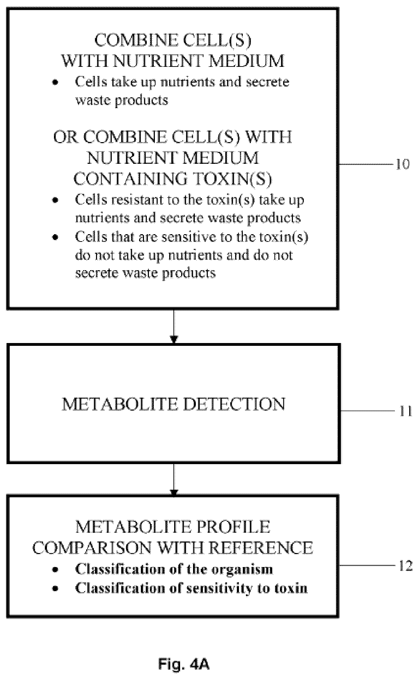

**Fig. 4A****.** Shows an example of a method flow diagram for identifying an unknown organism and determining its sensitivity to toxins.

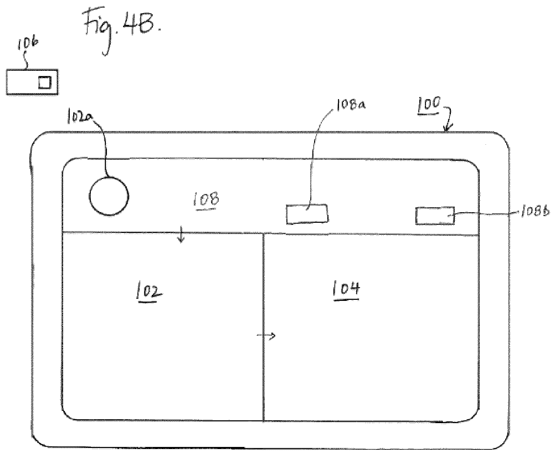

**Fig. 4B****.** Schematically illustrates a device according to the invention.

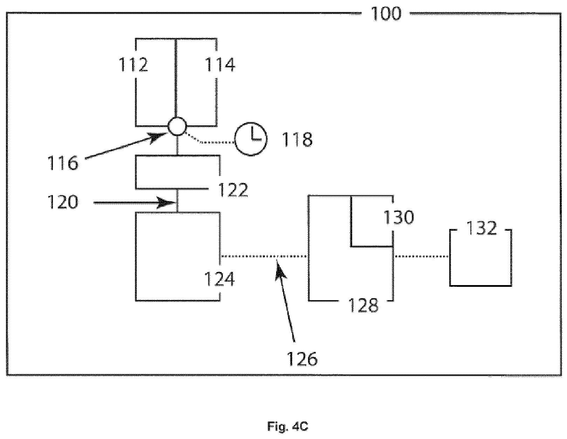

**Fig. 4C****.** Shows another embodiment of a device according to the invention.

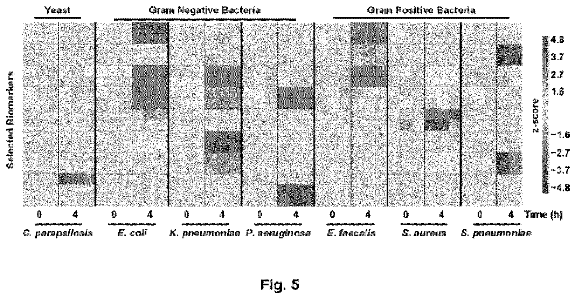

**Fig. 5****.** Shows metabolite-based identification of seven unknown organisms. Heat map of biomarkers (plotted as z-scores) selected from over 250 metabolites observed in the MS spectra. The biomarkers are plotted prior to and after a 4h incubation in a nutrient rich medium.

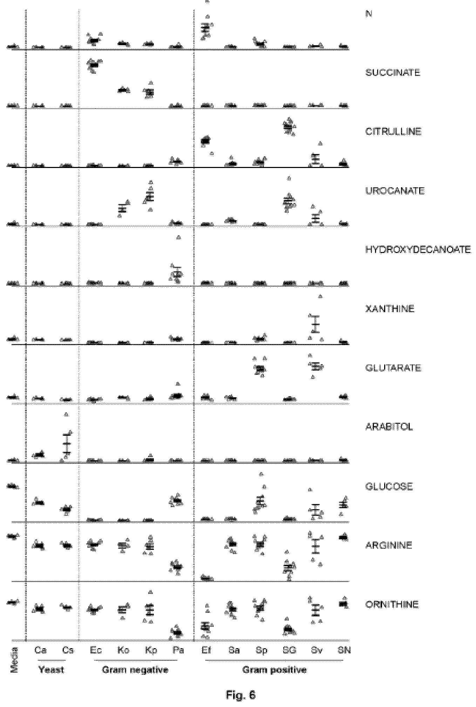

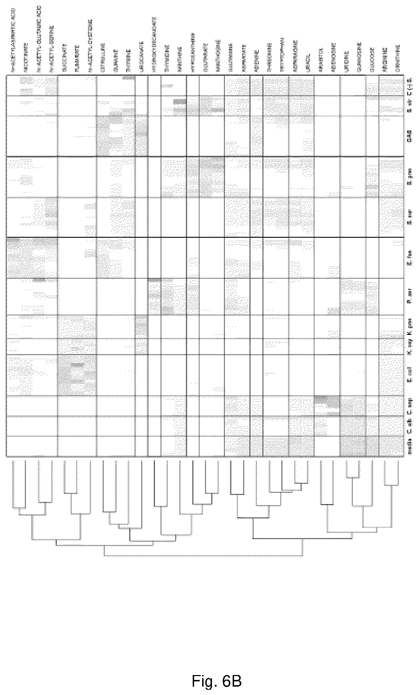

**Fig. 6****.** Shows selected biomarkers, as measured by MS, present in the media from microbial cultures. Cultures were standardized with 0.5 McFarland dilutions of common pathogens and commencal organisms. Sample key: *Candida* species, Ca, Cd, Cg, Ck, Cp; *Escherichia coli, EC; Klebsiella oxytoca, KO; Klebsiella pneumoniae, KP; Pseudomonas aeruginosa, PA; Pseudomonas putida, Pp; Staphylococcus aureus, SA; Enterococcus faecium, EF; Streptococcus pneumoniae, SP;* group A *Streptococcus, SG; Streptococcus viridans, SV; and coagulase negative Streptococcus, SN.*

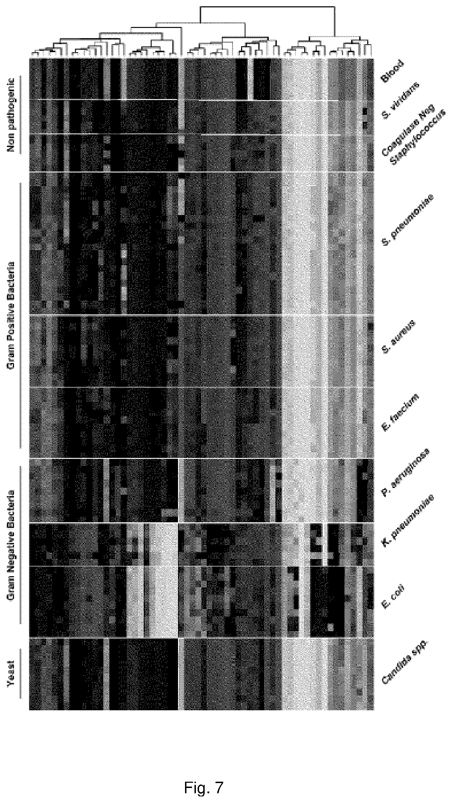

**Fig. 7****.** Shows selected metabolite levels observed in the growth medium of 100 cultures clinical isolates of bacteria. Each media sample was inoculated with 10⁸ bacteria per ml and incubated for four hours. Metabolite levels across the target organisms were then identified by mass spectrometry.

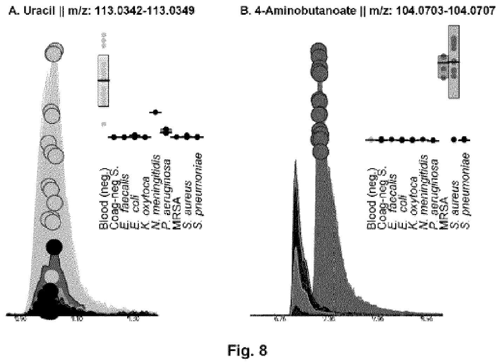

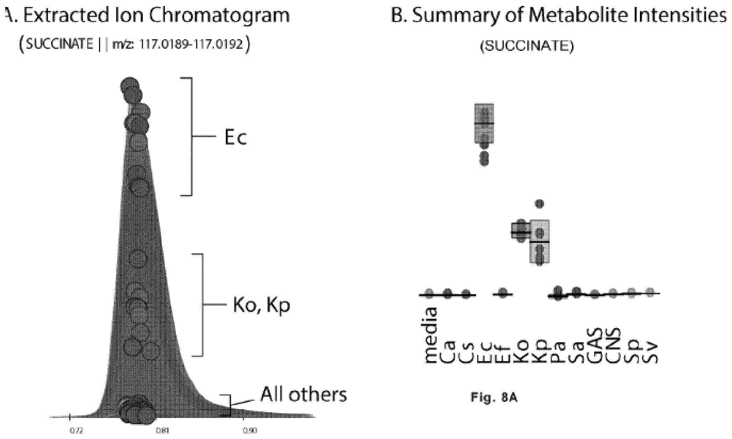

**Fig. 8****.** Shows select biomarkers detected in spent blood culture bottles from a clinical diagnostic laboratory as determined by MS.
**Fig. 8A**. (A) Shows a raw mass spectrometer dataset (extracted ion chromatogram) showing the signal for succinate observed in Mueller Hinton after four hours incubation in the presence of microorganisms. This figure depicts the diagnostic differences in Escherichia and Klebsiella signals versus those seen in nine other microbes. (B) The mass spectrometry intensities for succinate depicted as a boxplot. This figure depicts the same data as shown in Fig. 8A (A), but in a processed format.

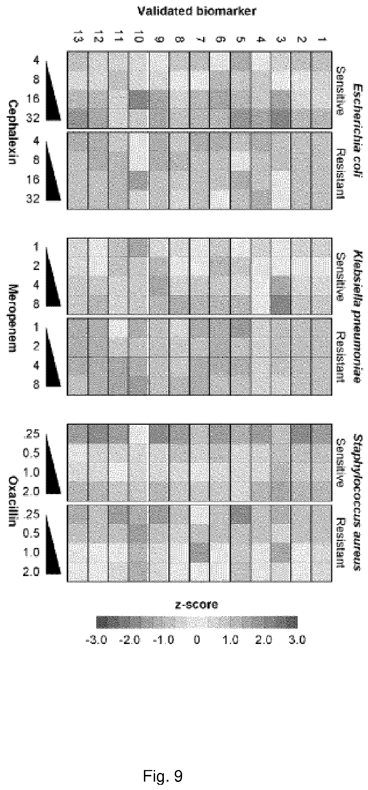

**Fig. 9****.** Shows biomarker panel of sensitive versus resistant strains of three target organisms. Drug doses are listed in µg/ml. A computer model using these biomarkers successfully differentiated the organisms.

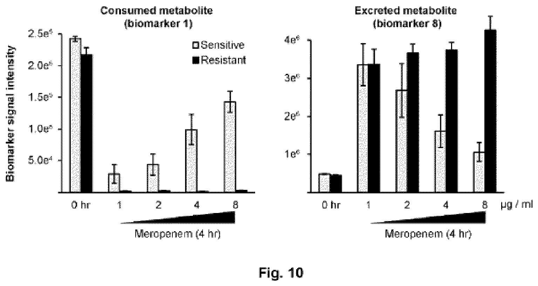

**Fig. 10****.** Shows metabolic detection of antibiotic resistance by mass spectrometry. Biomarker levels were assessed in sensitive and carbapenem-resistant *K*. *pneumoniae* across a range of meropenem doses. This data shows toxin-induced changes in metabolism that differentiate drug-sensitive and resistant isolates. This data is a sub-set of the larger set shown in Fig. 9.

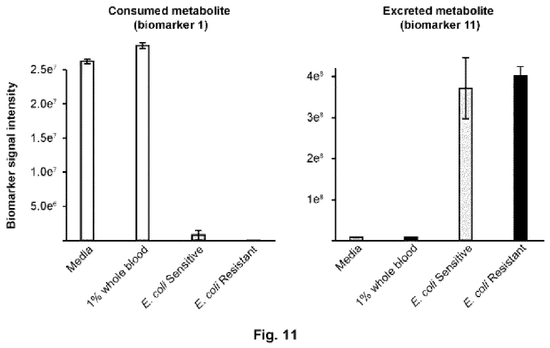

**Fig. 11****.** Shows the contribution that the human serum and cells may have on biomarker signals. Samples were spiked with 1% of whole human blood and were allowed to incubate for 4 hours.

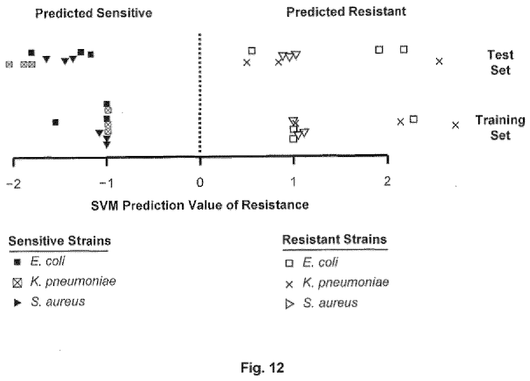

**Fig. 12****.** Shows computer prediction of drug sensitivity. Drug-induced changes in biomarker levels were recorded in 36 clinically-relevant strains (three species with sensitive and resistant isolates, 6 replicates; *E. coli* +/- extended-spectrum beta-lactamase, *K. pneumoniae +*/*-* carbapinem resistance, and *S*. *aureus* +/methicillin resistance). These biomarker levels were used to create a database training set. A computer model predicted resistance using biomarker levels in 18 samples.

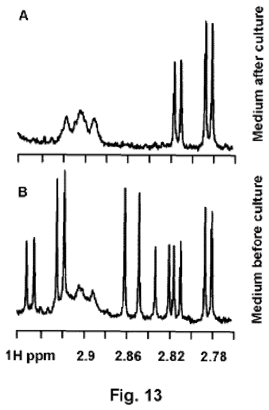

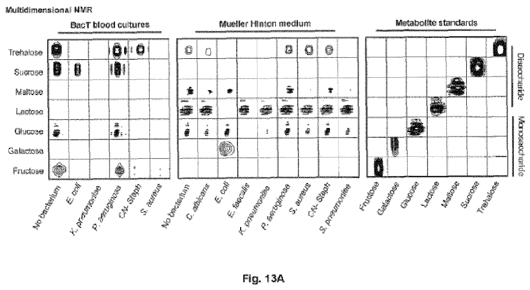

**Fig. 13****.** Shows select biomarkers detected in spent blood culture bottles from a clinical diagnostic laboratory as determined by ¹H NMR. In this example, the patient was suffering from a bloodstream infection with *Pseudomonas aeruginosa.* Nutrients missing from the growth medium result from the metabolic action of the pathogen.
**Fig. 13A****.** NMR was employed to analyze sugar monomers (saccharides and disaccharides) in two types of growth medium after incubation with common bacteria. The Figure shows diagnostic metabolite signals observed in two growth media as detected by multidimensional (1H-13C) nuclear magnetic resonance spectroscopy (NMR). Diagnostic NMR regions of interest corresponding to the each of seven target sugars are shown in growth media and in solutions of pure standards (100 mM). Bacterial isolates were grown for four hours in either BacT blood culture medium (BioMérieux) or Muller Hinton medium. The two media contain different carbohydrate nutrients as shown in the no bacterium control samples. Microbes grown in the two media produced diagnostic patterns of metabolites that were sufficient for differentiating the microorganisms (e.g. the presence of sucrose after the incubation in BacT medium distinguishes *Escherichia coli* from *Klebsiella pneumoniae*)*.*

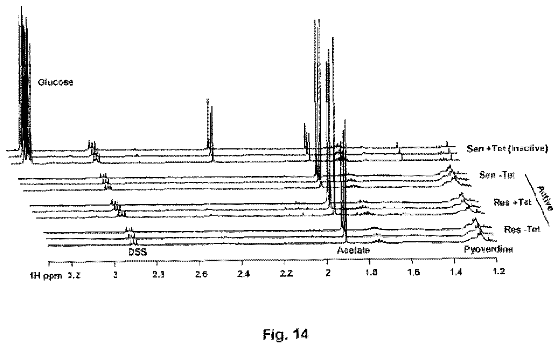

**Fig. 14****.** Shows ¹H NMR-based differentiation between drug sensitive (Sen) and drug-resistant (Res) isolates of *Pseudomonas aeruginosa* in the presence and absence of 60 µg/ml tetracycline (Tet). Metabolite biomarkers of drug efficacy are noted along with DSS, an internal standard.

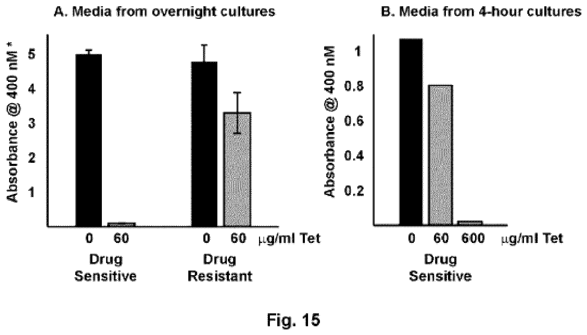

**Fig. 15**. Shows metabolic detection of antibiotic resistance by optical absorbance. Cultures of *Pseudomonas aeruginosa* were prepared in the optically-neutral M9 medium. The microbial production of the optically-active pyoverdine was detected by absorbance at 400 nM in media that had the cells removed via centrifugation. The optical differentiation of drug sensitive (Sen) and drug-resistant (Res) isolates of *Pseudomonas aeruginosa* in the presence of 0, 60, and 600 µg/ml tetracycline (Tet) is shown.

### Detailed Description

The detailed description and examples set forth below are intended as a description of various embodiments of the present invention and are not intended to represent the only embodiments contemplated by the inventor. The detailed description includes specific details for the purpose of providing a comprehensive understanding of the present invention. However, it will be apparent to those skilled in the art that the present invention may be practiced without these specific details.

The current practice of identifying microorganisms and determining their sensitivity to toxic substances (e.g. antibiotics) is as follows: (1) samples of biological fluids or tissue swabs are collected from a patient; (2) samples are combined with a nutrient-containing growth medium (either solid or liquid); (3) samples are incubated to allow microorganisms to grow until they reach detectable levels (approximately 18-48 hours); (4) microorganisms are identified based on protein profiles using chemical tests or spectrometric methods [e.g. matrix assisted laser ionization desorption (MALDI) mass spectrometry (MS)]; (5) aliquots of the microorganisms are placed in growth medium (either solid or liquid) containing toxin(s); (6) the growth rate of the microorganisms with and without the toxin(s) over approximately 18-48 hours is determined; and (7) data from reference microorganisms is used to determine toxin-sensitive versus toxin-resistant growth rates.

Living cells, such as microorganisms, continuously metabolize: take up nutrients and secrete waste products. Living cells, such as microorganisms, use metabolic nutrients from their environment to supply their energy, redox, and biosynthetic needs. These general requirements of life can be met via a variety of metabolic pathways. Microorganisms have developed diverse metabolic strategies for acquiring and processing their nutrients. These metabolic activities are constrained by the genetic composition and the environmental conditions of the organisms. Each molecule taken up by a microorganism contributes to a complex network of chemical reactions. Metabolic waste products from these networks are substances that do not contribute to the viability of the organism and are secreted to the environment. These metabolic endpoints depend on an organism's metabolic pathway architecture, which is constrained by genetic and environmental factors. Consequently, if environmental conditions are controlled, then the metabolites, which are molecules taken up by organisms and substances they secrete back to their environment, are biological markers for the genetic composition of the microorganism. These metabolites are detected by the present invention and are used to identify the cell type of the organism. The cell type of the organism may be the general class of the organism (i.e. gram negative, gram positive, etc.), the species or species of origin (i.e. the bacterial species, human, etc.), or the strain or the distinguishing characteristic (i.e. human blood cell, resistance or sensitivity to a toxin such as an antibiotic or chemotherapy, quiescence or actively growing, successfully genetically transformed, etc.).

The biological consumption of nutrients and the secretion of waste products is an essential component of living cells. Environmental conditions, such as the presence of toxins, may modulate a cell's metabolism by killing the cell or stimulating or substantially impairing the flow of metabolites into or out of the cell. The present invention, may also detect the cell type relating to a subspecies, strain characteristic such as its toxin-induced changes to nutrient uptake and waste secretion. Toxins may include, for example, antibiotics, inorganics, cancer chemotherapies, etc.

Growth medium in which cells are grown provides the nutrients and accumulates the waste. Consequently, the metabolic signal of microorganisms is amplified over time through cumulative changes in media composition. As a result, metabolites in the growth medium can be more than 500,000 times more abundant than the peptides and proteins currently used for MALDI-MS classification of microorganisms. Thus, a metabolically-based assay allows microorganisms to be detected at low concentrations. For example, the present invention can identify a microorganism based on analysis of a sample with fewer than 100 bacteria per milliliter (Fig. 3). This sensitivity shortens the incubation times compared to the current practice.

Also, in the present invention the metabolites of greatest interest are small molecules, for example, of less than 600 Daltons, or even less than 400 Daltons. Such metabolites are mostly monomers. These metabolites are consumed and appear very rapidly in the growth medium and, particularly, much more rapidly than macromolecules such as peptides and proteins currently used for MALDI-MS classification of microorganisms.

The devices, methods and systems of the present invention identify the cell type of an organism. It can identify an unknown organism's general class, species or particular cellular characteristics such as toxin sensitivity.

In one embodiment, a method (Fig. 4A) includes: incubation 10 of a sample in a growth medium, chemical analysis 11 of metabolite biomarkers in the growth medium after incubation; and identification 12 of a microorganism in the sample by comparison of metabolite biomarker levels in the growth medium with reference metabolite profiles.

The incubation period allows cells in the sample to metabolize: consume their preferred nutrient(s) and secrete metabolic waste products. Metabolites present in the medium after analysis are analyzed to obtain metabolic data for the organisms in the growth medium. Reference metabolite profiles, which are the known metabolite results for groups of microorganisms or individual species or subspecies and strains, as described below, are compared to the metabolic data acquired from analysis of the sample, thereby to classify the unknown organism.

Samples that can be added to the growth medium include, but are not limited to: food, tissue, biological fluids, such as for example any of feces, blood, urine or cerebral spinal fluid or swabs, such as from living or non-living surfaces (i.e. tissue swabs or swabs from clinical surfaces). The sample may be unprocessed or may be pretreated. In one embodiment, for example, the method includes pretreating the sample to separate microorganisms from remaining sample contents. For example, a swab may be soaked to collect microorganisms therefrom. As another embodiment, the method may include separating microorganisms from other sample constituents such as other cells. For example, blood samples may be processed to separate microorganisms from patient cells such as blood cells. This processing, for example, may be by size exclusion such as filtration or centrifugation. However, it is noted that experimental data has shown that the present method can accurately identify a microorganism from a sample, even where it contains other living cells such as a biome or blood cells.

To facilitate separation, the method may further include sample dilution. Thus, the method may include pretreating the sample including diluting the sample and separating the microorganism cells from the diluted sample. Dilution may be growth medium or nutrient-free wash solutions, such as sterile saline.

With or without separation, in another embodiment, a pretreatment step includes concentrating the microorganisms from the sample into a concentrated analysis solution. For example, concentrating can be by filtering or separation by density (i.e. centrifuge). Concentration can reduce the sample volume by 1/10 to 1/100,000. For example, a 1-10ml sample can be reduced to less than 50µl. For example samples of about 5-25µl are useful. Concentration may result in a microorganism concentration of less than 500 cells per ml or possibly less than 100 cells per ml. In one embodiment, the step or concentration brings the microorganisms to a known or desired cell count per volume (concentration).

The growth medium contains nutrients to support cellular metabolism. Because the present invention is based on the analysis of normal metabolism, the growth medium need not contain any non-typical biomarkers or macromolecules, but instead may be typical growth medium such as, for example, liquid or solid formulations of M9, Mueller Hinton medium, Lysogeny broth, tryptic soy broth, yeast extract peptone dextrose, BacT^{™}, BacT/Alert^{™}, Vitec^{™}, Dulbecco Modified Eagle Medium^{™} or Roswell Park Memorial Institute^{™} medium. In one embodiment, a growth medium is used that has a custom composition to support growth of one or more selected microorganisms, according to the nutrient requirements of the one or more selected microorganisms. This may control the particular cell to be cultured and/or may simplify analysis, as there will be fewer metabolites to identify. In one embodiment, a control chemical that is not involved in metabolism may be added to the growth medium for tracking use during spectrometric analysis. In one embodiment, isotope labelling can be employed to permit tracking. For example, a known nutrient may be labelled so that the metabolism and modification/secretion thereof can be followed.

Rich medium with many nutrient options may facilitate cell growth, to thereby increase the speed of analysis and thereby speed of cell type identification. On the other hand, simpler medium with fewer nutrients may readily select for the growth of fewer cell types but may be easier to analyse and thereby facilitate cell type identification.

In one embodiment, the metabolic profile of the unmodified growth medium, before adding the sample, is of interest for comparison and may be obtained by chemical analysis. In some methods, a control of the growth medium may be collected shortly after addition of the sample. Any microorganisms in the control may be killed to stop metabolism. In other embodiments, no control is required if the spectrometric profile is already available for a selected growth medium or the starting growth medium spectrometric profile is not of interest.

Incubation permits the microorganisms in the sample to metabolize to consume and generate metabolites. Incubation may be carried out at an elevated temperature such as about human body temperature for example 35-40°C. Incubation should be maintained for a period suitable to generate detectable amounts of waste products from metabolism. In one embodiment, the method includes incubation for 1-6 hours, such as 2-4.5 or 3.5-4.5 hours. In one embodiment, time of incubation is set and variance is limited to +/- 30 minutes or even lower such as +/- 10 minutes. In particular, microbes may undergo various stages of metabolism during their life cycle. During a first stage of metabolism certain first chemicals are generated and over time those first chemicals are broken down by further metabolism or natural decay. In situations such as the acidogenic to solventogenic shift, the metabolite profile may change over time. As such, it may be important to control the period of time for incubation in order to establish the metabolic profile of the sample at a particular stage of metabolism. Control of the duration of incubation may ensure the repeatability of the method and accuracy of the sample profiles as against reference profiles obtained from similarly timed incubations.

As noted hereinabove, it is desirable to reduce the time for identification of organisms. In the method, the incubation step may require the most time. To reduce the overall time for analysis, the method may include performing any pretreatment steps in growth medium and possibly also applying heat during the pretreatment steps. For example, ambient or heated growth medium can be employed for dilution, separation and concentration such that incubation is initiated and the microorganisms begin to metabolize during these steps. Also, equipment used for pretreatment can be heated. For example, the equipment, such as for dilution, filtration, centrifugation, etc., can be heated to about 35-40°C.

After incubation, the growth medium is analyzed to determine its metabolite content, which is its metabolic profile. In one embodiment, after a selected incubation time, the growth medium is quenched to stop metabolism. In other words, any living cells in the growth medium are killed. The method employed for quenching may be selected to reduce chemical modification, thereby to preserve the metabolites. In one embodiment, methanol is added to the growth medium to stop metabolism.

The method includes chemical analysis of the growth medium after incubation to identify the metabolites in the growth medium. In particular, the metabolites are employed as biomarkers and the levels of various metabolites are determined possibly including those consumed and produced.

Chemical analysis can be simple such as by pH assessment, analysis by a glucometer, simple chromatography or simple optical analysis. These methods are particularly useful for medium with simple profiles (Fig. 14) or where only a few metabolic biomarkers are of interest. While straightforward, the simple forms of chemical analysis may create a suitable signal indicative of the levels of the one or more metabolic biomarkers in the growth medium.

However, for more complex analysis, where, for example, the medium is more complex or more than one cell type may be present in the sample, more complex chemical analysis may be useful such as spectrometric analysis. Of course, the level of any particular metabolite is indicated by spectrometric signal intensity. The actual concentration of a biomarker is not necessarily determined, but as will be appreciated the spectrometric data is collected as a signal that has an intensity that may correlate to a concentration, all of which is referred herein as a metabolite level. When the growth medium is spectrometrically analyzed, a signal is generated that indicates intensities of a number of biomarkers. Because of the unique metabolic system of each type of microorganism, the growth medium from each species of microorganism generates a unique signal when the data from one or more metabolic biomarkers is considered. The resulting spectrographic data regarding the levels of one or more biomarkers is termed a metabolic profile. Spectrometric analysis may be by mass spectroscopy (MS), nuclear magnetic resonance spectroscopy (NMR) or spectrophotometry such as by optical analysis. Some useful MS platforms are liquid chromatography MS (LC-MS), triple quadrapole MS or high resolution MS.

Once analyzed, the microorganism can be identified by comparing the sample's resulting metabolic profile (i.e. spectrometric data regarding the biomarker levels in the growth medium after incubation) against reference metabolic profiles for known microorganisms grown in similar medium over a similar incubation period. As will be apparent from the examples that follow, such comparison can be done manually.

However, for speed of analysis the data can be analyzed by an analog, electronic or computerized processor against, for example, a stored database of reference metabolic profiles. Using a computer system with software, for example, numerous metabolite reference profiles may be rapidily compared to the metabolic data acquired from the sample to identify the cell type in the sample.

It is not necessary to identify the metabolites used as biomarkers provided the analysis data is compared against a reference metabolic profile obtained from similar conditions of growth medium, cell concentration and time period for incubation. Reproducible spectral features can be obtained by molecules that are related through structure or metabolic function such as amino acids, nucleosides, carbohydrates, tricarboxylic acid cycle intermediates and fatty acids.

As noted, metabolic molecules of greatest interest are those that are readily consumed or formed by cellular metabolism such as simple carbohydrates, amino acids, nucleobases and their derivatives. Such molecules are often smaller than 600 or 400 Daltons and are monomers or simple complexes of two or three monomers.

Diagnostic metabolites observed in microbial cultures are frequently excreted with closely related molecules originating from the same metabolic pathway. As shown in Fig. 13A, incubating E. *coli* in the presence of lactose will result in the production of both glucose and galactose, which are the breakdown products of lactose. Thus, the presence of any metabolite from this pathway can be used to diagnose the presence of *E*. *coli* under the conditions used in this study. Similarly, inosine, hypoxanthine, xanthine, guanine, inosine monophosphate, xanthosine monophosphate, and uric acid are all metabolites that can be derived from guanosine monophosphate and reflect the action of a shared metabolic pathway. More broadly, the presence of diagnostic nucleotides, or their break down products, in the growth medium is indicative of a specific microbial metabolic activity that can be used as a diagnostic indicator for the type of microorganism. Similarly, amino acid breakdown products originating from a specific metabolic activity (e.g. arginine catabolism) communicate overlapping diagnostic information. For example, agmatine, putrescine and ornithine, each originate from the breakdown of arginine and can be used as diagnostic indicators of microorganisms. Classes of molecules where metabolic pathway activity results in clusters of closely related diagnostic metabolites include carbohydrate metabolism (e.g. Fig 13A, glucose and galactose from lactose), nucleotide metabolism, amino acid metabolism, tricarboxylic acid cycle metabolism, and fatty acid metabolism.

Specific metabolites that have been identified as useful for the identification of 85% or more of the pathogens of clinical interest are adenine, adenosine, arginine, 4-aminobutyrate, cytidine, glucose, glutarate, glycine, guanine, guanosine, hypoxanthine, inosine, N-acetyl-phenylalanine, ornithine, sn-glycerol-3-phosphate, succinate, taurine, uridine, urocanate and xanthine or derivatives thereof. There are also nine metabolites that have not been identified but are seen in spectrometric analysis and are useful to differentiate microorganisms. No more than these 30 molecules are needed to correctly identify the following eight microorganisms: *Escherichia coli; Klebsiella pneumoniae; Klebsiella oxytoca; Pseudomonas aeruginosa; Staphylococcus aureus; Enterococcus faecium; Streptococcus pneumoniae;* and *Candida parapsilosis,* which cause more than 85% of human bloodstream infections.

By analysis of the growth medium after incubation, metabolic differences are detected, thereby to permit identification of the cell type being incubated. For example, with reference to Fig. 5 and Fig. 6, gram negative bacteria, such as *Escherichia coli* (EC) and *Klebsiella oxytoca* (KO), secrete diagnostic quantities of succinate and consume inosine when grown in Mueller Hinton medium. Under the same conditions, however, the gram negative *Pseudomonas aeruginosa* (PA) has little impact on succinate and inosine but consumes diagnostic quantities of ornithine. Similarly, the gram positive bacterium *Staphylococcus aureus* (SA) consumes diagnostic quantities of taurine and secretes N-acetyl phenylalanine when grown in Mueller Hinton whereas the gram positive *Enterococcus faecalis* (EF) consumes diagnostic quantities of arginine. Likewise, the gram positive *Streptococcus viridans* (SV) and *S*. *pyogenes* (SP) produce diagnostic levels of glutarate when incubated in Mueller Hinton whereas group A *Streptococcus* (SG) and coagulate negative *Streptococcus* (SN) do not.

Thus, the metabolite biomarkers are useful for identification of cell type, for example, the presence of broad classes, species or strains of cells. One or more biomarkers are specific to individual species and thus, the method can accurately identify cell type when considering the presence of the one or more biomarkers in growth medium incubated with a cell of unknown cell type.

One cell type of interest is the characterization of cell's sensitivity to a toxin. Thus, the method can also be used for analysis of a sample for toxin sensitivity of the cells therein. A similar method is employed, but the growth medium includes an amount of a toxin such as an antibiotic or a chemotherapy. With reference to the options noted above and Fig. 4A, the method includes combining a sample likely containing a microorganism with a growth medium and an amount of a toxin such as an antibiotic or chemotherapy, for example any one or more of amoxicillin, penicillin, tetracycline, vancomycin, streptomycin, cephalexin, erythromycin, clarithromycin, azithromycin, ciprofloxacin, levofloxacin, ofloxacin, chloramphenicol, bactrim, bacitracin, linezolid, cefepime, cefoperazone, cephalexin, meropenem, clotrimazole, econazole, azide, rotenone, antimycin A, chloroquine, nitazoxanide, melarsoprol, eflornithine, tinidazole, miltefosine, metronidazole, 5-fluoromuricil or deoxycytidine. The mixture of cell-containing sample, growth medium and toxin is incubated for a period of time. During the incubation period, organisms that are not sensitive to the toxin(s) may consume their preferred nutrient(s) and secrete metabolic waste products, whereas organisms that are sensitive to the toxin(s) may become metabolically perturbed or inactive. The growth medium after incubation is chemically analyzed 11 and metabolite levels is the growth medium are compared to reference metabolite profiles from cell cultures with toxin-induced metabolic changes to identify 12 the toxin-sensitivity of the microorganism in the sample. The comparison may be by various means such as manually or automatically. Using a computer software, for example, metabolite reference profiles may be readily compared to the metabolic data acquired from analysis of the incubated growth medium to classify the toxin sensitivity of the organism. This method may be used on its own to classify the toxin sensitivity of the organism. Alternately, this method may be employed to identify toxin-sensitivity after sequentially or in parallel with a method to identify the microorganism in the sample.

Thus, the present method may be used to detect individual cell types in a sample, such as the bacterial species and/or drug sensitivities of a microorganism organisms present in a sample. The method may be useful to differentiate between two or more microorganisms. The present invention may also be used to identify cell types in mixtures of cell species or the one or more drug sensitivities of one or more organisms present in a sample.

The present invention may be used to analyze samples originating from a single sample or a single patient. The present invention may also be used to acquire data on multiplexed samples originating from a plurality of samples or a plurality of patients.

The method may include operating an analytical device to carry out one or more steps of the method. For example, the growth medium after incubation may be loaded into an analytical device for analysis and comparison. Alternately the method may include loading the sample into the device and the method is carried out entirely in the device.

As schematically illustrated in Fig. 4B, a device 100 according to the invention includes at least two components: an analytical data acquisition tool 102, and a computer system 104 that compares output results from analytical data acquisition tool 102 with reference standards, reference metabolite profiles, to identify a cell's type, such as its broad class, its species or a cell characteristic, such as strain, toxin sensitivity or verification of recombination.

Analytical data acquisition tool 102 may be one based on simple chemical analysis such as pH, electrical conductivity or the presence of glucose or a more complex technology such as one based on spectrometry such as for example, mass spectroscopy ("MS"), nuclear magnetic resonance spectroscopy ("NMR") or spectrophotometry such as by optical analysis. Tool 102 includes an inlet port 102a for accepting an amount of growth medium for analysis thereof. The device may be configured to handle unprocessed or processed growth medium. When considering processed growth medium, the device may be configured to accept and handle packaged growth medium 106 such as on a cassette or strips or in tubes, gels, etc.

Device 100 also includes computer system 104 in communication with tool 102 and configured to receive results from tool 102. System 104 further includes a processor configured to analyse the data from tool 102 and to identify the cell type such as broad class, species and/or strain/cellular characteristic such as toxin resistance. In one embodiment, the data is a metabolite profile and the computer system includes a computer storage element for storing a database of reference metabolite profiles.

Multiple reference metabolite profiles are used to populate the database and enable the computer system. The computer model compares the information received from the sample testing with reference metabolite profiles to determine the identity of the unknown organism and/or its sensitivity to toxins. A computer system, for example software, compares the data acquired from the sample to reference metabolite profiles. Samples are classified by a level, such as presence, absence or amount above or below a threshold or within a specified range, for a single biomarker or a plurality of biomarker levels. The biomarker level (i.e. presence, absence or level) is indicated by spectrometric signal intensity. The reference metabolite profile can include reference data for a selected one or more biomarkers in a specified growth medium and general cell concentration, for a class of or specified microorganism and after a specified incubation period. Alternately, the reference metabolite profiles are each a cumulative spectrometric signal or pattern across a spectrum for a class of or specified microorganism at a cell concentration in a specified growth medium, after a specified incubation period. The data from an analyzed growth medium can be matched to a reference metabolite profile using simple processing or an algorithm (e.g. support vector machine, principle component analysis, single value decomposition).

In one embodiment for complex analysis, the device comprises a support vector machine algorithm that may be used to automatically classify microorganisms from clinical samples and distinguish drug-sensitive versus resistant strains of microorganisms.

In order to establish a database of reference metabolite profiles, known organism can be incubated under known conditions of nutrient source, time and toxin concentration. The growth medium after such an incubation can be analyzed and the resulting data recorded for one or more natural metabolite biomarkers such as the up to 21 or 30 biomarkers noted above or the full signal may be recorded across a spectrometric spectrum, and this data can be recorded as a reference metabolic profile. When reference metabolic profiles are obtained from a plurality of cells, such as microorganisms, of interest, this data can be stored in the computer system to create a database and support a fully automated computer program, useful to identify unknown organisms and classify sensitivity to toxin(s) based on the metabolic profiles obtained from clinical samples. The reference data can readily be applied to resolve signals from samples containing one or more types of microorganisms.

The computer system is configured to output information regarding an identification of the microorganism in the sample and possibly any toxin resistance.

In another embodiment, the device may include a sample pretreatment chamber and/or an incubation chamber 108. Input port 102a would then provide sample input to these chambers. These chambers may contain supplies of growth medium, dilution liquid, etc. such that the device is self-contained and configured to carry out a method from receiving an unprocessed sample to microbe identification. A sample pretreatment chamber may be configured for processing an unprocessed sample to a form suitable for incubation. For example, the sample pretreatment chamber may include dilution and size exclusion, such as filtration apparatus 108a. The incubation chamber and possibly the sample pretreatment chamber includes a heater 108b.

Fig. 4C shows another embodiment of a device 100 according to the invention. Device 100 of Fig. 4C includes a first microbial incubation chamber 112, a second microbial growth chamber 114 and a sample preparation chamber 122. A sampling port 116 provides communication from the chambers 112, 114 to a sample transfer tube 120, which opens into chamber 122. A timing device 118 controls the operation of sampling port 116 so it only opens when permitted by the timing device. Incubation time in chambers 112, 114 can be controlled by the timing device.

The device further includes an analytical metabolite data acquisition device 124. A tube 120 leads from chamber 122 to device 124.

Analysis device 124 is connected by a physical or wireless communication 126 to a data analysis device 128 that operates with a database of reference metabolic profiles data analysis device 128. Data analysis device 128 outputs to a display device 132, such as a printer, a screen, a computer terminal, etc. on the device or coupled wirelessly or through physical connections to the data analysis device of device 100.

In use, microbial samples and growth media are added to a microbial incubation chamber 112. In parallel, or sequentially, samples from chamber 112 are transferred to second microbial growth chamber 114 where the sample-containing growth medium may be mixed with a toxin 114. Cultures in chambers 112 and/or 114 are incubated for a fixed period of time by way of a timing device 118. Device 118 controls the operation of sampling port 116.

Culture samples are transferred from chambers 112 and/or 114 through the sampling port and sample transfer tube 120 and are delivered to sample preparation chamber 122.

After preparation, samples are transferred from the preparation chamber to a metabolite analysis device 124 and signals of metabolite levels are generated. Observed metabolite signals are transmitted to a data analysis device 128 that uses a reference dataset 130 and processor to identify the cell type or plurality of cell types present in the microbial sample. The organism type is then reported via the display device 132.

After loading the sample, the device can provide an output of the organism type in 2 to 8 hours.

In another embodiment, the invention relates to a treatment regimen for treatment of an infection comprising one or more aspects of the methods described above and identifying a toxin for acting against the cell type identified. In another embodiment, a method for treating an infection that comprises one or more aspects of the method described above and further administering an effective amount and type of antibiotic(s) to a patient suffering from infections based on the identification of a microorganism and its sensitivity to antibiotics using metabolite data acquired. As such, one or more aspects of the method described above and further selecting a type of antibiotic based on the identification of a microorganism and its sensitivity to antibiotics using metabolite data acquired, may be used for treatment of a patient suffering from an infection.

In another embodiment, a method according to the invention includes screening the effectiveness of a chemotherapy against a cancer cell using one or more aspects of the method described above, wherein a chemotherapy drug is added to the growth medium.

In another embodiment, a method according to the invention includes screening the effectiveness of a genetic recombination on a cell using one or more aspects of the method described above, wherein the recombination modulated metabolism and the method rapidly confirms that the recombination was successful.

In another embodiment, the present invention is a method for determining whether a patient has an infection, comprising: obtaining a patient specimen (e.g. blood, urine, swab, stool) or a clinical specimen (i.e. hospital equipment swab); combining the specimen with a growth medium; data acquisition of the organism's metabolic activity; diagnosing the patient as having an infection based on metabolite biomarkers concentration changes or are present relative to reference metabolite profiles. The method may further include suggesting a treatment or administering the appropriate antibiotic and dose of antibiotic relative to the concentration of metabolite present.

In another embodiment, the method of the present invention, as described above, is determining whether a food is contaminated with a microorganism.

The following examples are included for the purposes of illustration only, and are not intended to limit the scope of the invention or claims.

### Examples:

### Example I: MS detection of the organism

The growth medium used in this example, Mueller-Hinton, enabled metabolite uptake by the microorganisms. This medium is prepared from 2 g beef extract, 17.5 g casein hydrolysate, and 1.5 g starch dissolved in 1 liter of deionized water. A microorganism-containing sample was mixed with the growth medium, the consumed and the produced biomarkers were evaluated using a diagnostic data collection tool. Microbial samples were prepared by making 0.5 McFarland standard dilutions (approximately 1 x 10⁸) of seven opportunistic pathogens: *Escherichia coli, EC; Klebsiella pneumoniae, KP; Pseudomonas aeruginosa, PA; Staphylococcus aureus, SA; Enterococcus faecium, EF; Streptococcus pneumoniae, SP; and Candida parapsilosis, CA.* These seven target microbes were selected because they cause more than 85% of the human bloodstream infections. At time 0 hours, standardized microbial samples were combined 1:1 with a Mueller-Hinton growth medium. Aliquots of each sample (100 microliters) were immediately harvested and metabolism was quenched by combining the aliquot with an equal volume of methanol and stored at 4°C until the data acquisition of the metabolic composition was available. An additional microbial aliquot was allowed to incubate in its growth medium at 37°C for four hours. At time 4 hours, samples were harvested (100 microliters), combined with an equal volume of methanol to quench metabolism and transferred to 4°C until the data acquisition of the metabolic composition was available. The microbial culture experiment was completed three times to generate three independent biological replicates. All samples were then analyzed by liquid chromatography mass spectrometry (LC-MS) using a hydrophobic interaction liquid chromatography column and a high-resolution mass analyzer acquiring data in both positive and negative ionization mode. Over 250 metabolites were analyzed by LC-MS and retention times and ionization properties were verified using metabolite standards analyzed on the same LC-MS platform. Both known metabolites (as defined by co-elution of observed signals with a reference standard using extracted ion chromatograms with 5 ppm mass windows) and unknown metabolites were identified and metabolite intensities were determined. Metabolite levels before and after the four-hour incubation were analyzed and 30 metabolites have proven to be sufficient in order to unambiguously differentiate between seven different target microbes. These metabolites are adenine, adenosine, arginine, 4-aminobutyrate, cytidine, glucose, glutarate, glycine, guanine, guanosine, hypoxanthine, inosine, N-acetyl-phenylalanine, ornithine, sn-glycerol-3-phosphate, succinate, taurine, uridine, urocanate, xanthine and 9 signals from unknown metabolites. From this preliminary experiment, it was concluded that the pattern of metabolites in the microbial growth medium could be detected by LC-MS and used to differentiate between common microbial pathogens.

### Example II: Automated detection of clinical isolates

To determine the diagnostic feasibility of the present invention, 100 microbial cultures of clinical isolates were prepared and analyzed as per the procedure in Example 1. The 100 isolates were prepared from nine groups of organisms (Fig. 6) representing common pathogens and commensal organisms observed in clinical diagnostic laboratories. Data from 250 known metabolites and all unknown signals were acquired. The 60 most statistically significant signals observed after 4 hours of incubation were determined by one-way analysis of variance ("ANOVA"). Hierarchical clustering of these selected biomarkers showed distinct species-related clustering in metabolite levels (Fig. 7). All 60 of these diagnostic signals were used to create a support vector machine (SVM) model of microbial species. A total of 21 samples were used to construct the SVM computer system and the microorganisms present in the remaining 79 clinical samples were predicted using the metabolite levels. The SVM computer system correctly identified the organism in all 79 blinded clinical samples. In addition, none of the samples were misidentified in this analysis, indicating a sensitivity >99% with <1% false discovery. This experiment indicated that a fully automated computer system based on analysing metabolite levels in the medium could correctly identify pathogens and common commensal organisms from a representative transect of clinical isolates.

Table 1 shows clinical isolates identified by an automated computer analysis of metabolite levels. Classifications were completed as per the procedure in Example 2. Numbers of correctly-identified organisms are shown - all organisms in this study were properly identified via a SVM computer system of metabolite levels observed after 4 hours of incubation in Mueller-Hinton growth medium. *Candida sp.* indicate diverse species from the yeast genus *Candida; VRE E. faecium* indicates vancomycin-resistant enterococcus. From this study it can be concluded that automated data acquisition of metabolite levels in growth medium are a feasible mechanism for performing diagnostic evaluation of clinical microbiology samples.

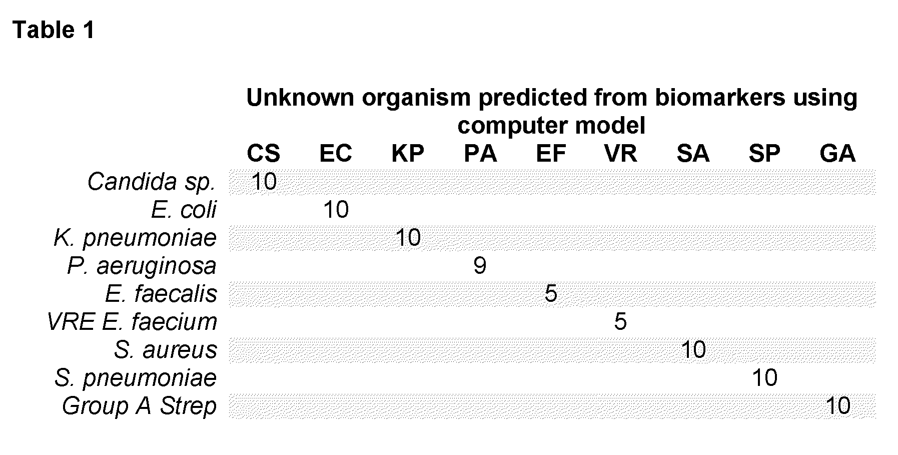

### Example III: Analysis of sensitivity limits

To ensure compatibility of the present invention with the clinical implementation, the analytical sensitivity of the device was measured. Cultures of *Pseudomonas aeruginosa* were grown to approximately 0.5 McFarland. The culture was then diluted using consecutive 1:10 dilutions in metabolite-free phosphate-buffered saline over a 5 orders of magnitude. The limit of detection for metabolite-based analyses was then determined by LC-MS as per the procedure in Example 1 and compared to traditional optical analysis of light scattering by bacterial cells at 600 nM. The LC-MS based assay showed a wider dynamic range and a lower limit of detection (< 100 cells / ml) as compared to the optical method. This example indicates that the device and LC-MS analysis has sufficient sensitivity for clinical application of the device.

### Example IV: Automated detection of antibiotic susceptibility.

Identifying drug-induced changes in metabolite levels could enable more rapid diagnostic assays. To determine the feasibility of this approach, microbial cultures were prepared, analysed, and classified as per the procedure in Example 1 except that the Mueller-Hinton growth medium in Example 1 was supplemented with a range of antibiotic concentrations (Fig. 9-10). Drug sensitive and resistant strains underwent clinical screening using the standard drug doses used by diagnostic laboratories. The pattern of metabolites taken up and secreted into the medium across the range of antibiotic doses matched the established minimum inhibitory concentrations ("MIC") for each of the bacteria (Fig. 9). Moreover, despite the presence of 1% blood intentionally added to samples to mimic blood culture applications, there was no overlap in background metabolite signals from the blood and the diagnostic signals from the microbial metabolism (Fig. 11). This indicates the compatibility of metabolite-based drug sensitivity system with clinical applications of this technology.

To determine if automated metabolite analyses could be used to detected microbial drug sensitivity, drug-induced changes in biomarker levels were recorded in 36 clinically-relevant strains (three species with sensitive and resistant isolates, each with 6 replicates; *E. coli* +/- extended-spectrum beta-lactamase, *K. pneumoniae* +/- carbapinem resistance, and *S*. *aureus* +/methicillin resistance). A computer model (SVM) was constructed to predict resistance using biomarker levels. Resistance levels were then predicted in 18 blinded test samples. The computer model correctly identified all resistant strains (Fig. 12). This study showed that toxin-induced changes in metabolite levels can be detected and used to automatically classify antibiotic sensitivity in clinically-relevant species.

### Example V: Clinical evaluation of metabolite-based microbial detection

To determine the compatibility of the metabolically-based microbial detection system, human blood cultures were collected directly from a clinical diagnostic laboratory and analyzed by MS. The existing VITEK (BioMerieux) culture system used for high-volume blood-borne pathogen testing platform functions by combining clinical blood specimens with a microbial growth medium. This is done to enable microbial growth for the downstream protein analysis used in the current technology (Fig. 8). Once bacterial densities have reached detectable levels (approximately 1,000 cells/ml) an aliquot is harvested and the bottles are discarded. These discarded blood cultures were collected directly from the clinical sample stream and analyzed by the present metabolite-based detection platform. Microbial metabolism was quenched by combining a 100 microliter aliquot with an equal volume of methanol, insoluble components were removed by centrifugation, and soluble extracts were analyzed by LC-MS. LC-MS analyses showed both general biomarkers of infection that differentiated positive from negative cultures and species-specific biomarkers similar to those seen in Examples 1, 2, and 3 (Fig. 5-7). This study showed that metabolic detection technology could be directly integrated into the existing clinical workflow.

This study also shows that blood metabolites and pathogen metabolites can be detected such that even samples with mixtures of cells can be analyzed and cells identified therein. Identification of a mixture of cells is also shown in Fig. 8A.

### Example VI: Detection of organisms by NMR and determination of drug sensitivity

Although LC-MS analyses is a powerful approach for detecting changes in the metabolic composition of growth medium, it is only one example of a wide range of possible analytical techniques that could be used to detect the metabolism of microorganisms. To determine the feasibility of NMR-based microbial detection systems, a clinical blood sample was obtained and prepared as per the procedure in Example V. The extract was then dried to remove the methanol component, resuspended in 100% D₂O containing 500 µM 4,4-dimethyl-4-silapentane-1-sulfonic acid (DSS; an internal standard used to reference chemical shifts). ¹H NMR spectra of the media were acquired on a 600 MHz instrument of a *Pseudomonas aeruginosa* positive culture. These data showed metabolic changes caused by the *P*. *aeruginosa* (Fig. 13).

These studies indicate that NMR has sufficient sensitivity to detect microbial metabolic activity in samples taken directly from the existing clinical pipeline.

To determine if NMR could be used to distinguish microorganisms, cultures of eight different microorganisms (*C*. *albicans, E. coli, K. pneumoniae, E. faecalis, P. aeruginosa,* coagulase negative *Staphylococcus, S. pneumoniae,* and *S*. *aureus*) were inoculated into in BacT blood culture medium (BioMérieux) or Mueller Hinton medium and grown for four hours. Metabolites were extracted as described above (Example VI) and analyzed by multidimensional 1H-13C heteronuclear single quantum coherence (HSQC) NMR. Diagnostic regions of interest in the NMR spectra that correspond to seven target sugars were extracted and compared to reference signals of metabolites standards prepared at 100 mM. The pattern of sugars observed in the growth medium was sufficient to distinguish all the target pathogens. This study indicated that NMR is capable of differentiating between types of microorganisms.

To determine if NMR could be used to detect antibiotic-induced perturbation in microbial metabolism, cultures of tetracycline sensitive and resistant isolates of *Pseudomonas aeruginosa* were prepared as per the procedure in Example 1. The Mueller-Hinton growth medium was substituted for M9 medium (47.7 mM Na2HPO4, 22 mM KH2PO4, 8.6 mM NaCL, 18.7 mM NH4Cl, 22 mM glucose, 2 µM MgSO4 and 100 nM CaCl2). Growth medium was prepared with and without 60 ug/ml tetracycline and were incubated with each strain for 12 hours. NMR samples of the media were prepared and analyzed as described above. The NMR data showed that all of the drug resistant isolates as well as the drug-sensitive isolate incubated in non-antibiotic medium were metabolically active; each active strain consumed glucose and produced acetate and pyoverdine (Fig. 14). In contrast, NMR analysis of the drug sensitive line incubated with tetracycline showed metabolic inactivation (i.e. minimal glucose consumption and minimal acetate and pyoverdine production). This study indicated that NMR is capable of detecting drug-induced inhibition in microbial metabolism.

While the analysis of Fig. 14 is by NMR, the simplicity of the system including the use of a simple growth medium with only a limited number of nutrients, lends itself as well to more simple chemical analysis. For example, the results to identify drug resistant isolates could be obtained with a glucometer. When simpler medium options are employed, growth may be slower, but growth medium analysis and identification against reference profiles may be facilitated.

### Example VII: Determination of drug sensitivity by spectrophotometry

Although NMR and MS offer opportunities for decoding complex mixtures of metabolites, optically engineered growth medium could also provide a mechanism for identifying microorganisms and measuring their sensitivity to toxin(s). To determine the feasibility of using optically-based methods for detecting drug-induced perturbations in microbial metabolism, *Pseudomonas aeruginosa* cultures were prepared as described in Example V. M9 growth medium, as described in Example VI, was used for this example because of its minimal optical properties, which enables sensitive detection of optically active metabolic waste products. Drug sensitive and resistant strains were incubated for either 4 or 8 hours in M9 medium containing 0, 60, and 600 µg/ml tetracycline. The cells were then removed by centrifugation and the cell-free medium composition was analyzed by spectrophotometric absorbance at 400 nM. The pyoverdine secreted by P. *aeruginosa* absorbs at this wavelength and can be used as a marker for metabolic activity (Fig. 15). Drug sensitive isolates showed impaired pyoverdine production when incubated with tetracycline, whereas the drug resistant lines did not. Moreover, the impairment in pyoverdine secretion was proportional to the concentration of the tetracycline (Fig. 15). This study showed that spectrophotometric analysis could be used to detect microbes and measure drug sensitivity.

### Example VII: Metabolite identifiers for common pathogens

A panel of the following organisms was analyzed by mass spectrometry after incubation in Mueller Hinton medium:
*Candida albicans, Candida* ssp (other species of *Candida*)*, Escherichia coli, Klebsiella oxytoca, Klebsiella pneumoniae, Pseudomonas aeruginosa, Enterococcus faecium, Staphylococcus aureus, Streptococcus pneumoniae,* Group A *Streptococcus, Streptococcus viridans,* Coagulase-Negative Staphylococci (CN-Staph).

The following metabolites were identified that can be used for identification and saved as reference metabolic profiles:
- Succinate levels differentiate *E*. *coli* and *Klebsiella* ssp from all other organisms in the panel;
- Urocanate levels differentiate *E*. *coli* and *Klebsiella* ssp;
- Hydroxydecanoate differentiates *Pseudomonas aeruginosa* from all others in panel;
- Arbitol levels differentiate yeast from all other organisms in the panel;
- Glucose levels differentiate *Pseudomonas aeruginosa* from all other gram negative organisms;
- N-Acetyl-Aspartate levels differentiate *Enterococcus* from all other organisms in panel;
- Xanthine differentiates *viridans* streptococci from others in the panel;
- The presence of galactose is diagnostic for *E*. *coli;* and
- Glucose versus lactose levels differentiate *E*. *faecalis* from other gram positive organisms.

A panel of organisms was analyzed by NMR after incubation in BacT medium:
- Sucrose levels differentiate *E*. *coli, Klebsiella* ssp and *S*. *aureus;* and
- Trehalose levels differentiate CN-*Staph* from *S*. *aureus* and *E*. *coli.*

The previous description and examples are to enable the person of skill to better understand the invention. The invention is not be limited by the description and examples but instead given a broad interpretation based on the claims to follow.

The invention covers the following items:
1. A method for identifying a cell type of a cell in a sample, comprising:
   incubating the sample in a growth medium;
   after incubation, analysing the incubated growth medium by chemical analysis to determine a level of each of one or more metabolites in the incubated growth medium; and
   identifying the cell type of the cell by comparison of the level of each of the one or more metabolites with reference metabolite profiles and matching the level of each of the one or more metabolites with a reference metabolite profile indicative of the cell type.
2. The method of item 1 wherein the cell type is toxin resistance and incubating is in the presence of the toxin.
3. The method of item 1 further comprising determining resistance of the cell to a toxin, wherein after identifying the cell type, the cell is incubated in a toxin-containing growth medium;
   analyzing the incubated toxin-containing growth medium by chemical analysis to determine a toxin-affected level of one or more further metabolites in the incubated toxin-containing growth medium; and
   determining resistance of the cell to the toxin by comparison of the toxin-affected levels of the one or more further metabolites with the reference metabolite profiles for the cell that are indicative of toxin-resistance.
4. The method of any one of items 1 - 3 further comprising: pretreating the sample including: diluting the sample and separating the cell from at least some other sample contents.
5. The method of any one of items 1 - 4 further comprising: concentrating the sample by reducing the volume by 1/10 to 1/100,000.
6. The method of items 4 or 5 wherein the sample is combined with the growth medium during at least some portions of pretreating and concentrating and the growth medium has a temperature above ambient during at least some portions of pretreating and concentrating.
7. The method of any one of items 1 - 6 wherein incubating is carried out for a selected period of time and the reference metabolite profiles are based on similar incubation times.
8. The method of any one of items 1 - 6 wherein identifying includes identifying the cell type as one of: *Escherichia coli; Klebsiella pneumoniae; Klebsiella oxytoca; Pseudomonas aeruginosa; Staphylococcus aureus; Enterococcus faecium; Streptococcus pneumoniae;* and *Candida parapsilosis.*
9. The method of any one of items 1 to 8, wherein the one or more metabolites are selected from: adenine, adenosine, arginine, 4-aminobutyrate, cytidine, glucose, glutarate, glycine, guanine, guanosine, hypoxanthine, inosine, N-acetyl-phenylalanine, ornithine, sn-glycerol-3-phosphate, succinate, taurine, uridine, urocanate or xanthine.
10. The method of any one of items 1 - 9 wherein:
   the growth medium includes glucose, inosine and ornithine and the one or more metabolites includes at least pyoverdine, acetate, inosine and omithine; and
   identifying identifies the cell type as *Pseudomonas aeruginosa.*
11. The method of any one of items 1 - 9 wherein:
   the growth medium includes inosine and ornithine and the one or more metabolites includes at least succinate, inosine and ornithine; and
   identifying includes differentiating the cell type as between *Escherichia coli* and *Pseudomonas aeruginosa.*
12. A computer system for identifying a cell type from a clinical sample, the computer system configured to carry out a method comprising:
   receiving a spectrometric signal indicative of a level of each of a plurality of metabolites in the clinical sample; and
   comparing the levels of metabolites with a database of reference metabolite profiles;
   matching the levels of metabolites with a reference metabolite profile indicative of the cell type; and
   outputting the cell type for a user.
13. The computer system of item 12 configured to carry out the method further comprising: receiving input of a characteristic of the clinical sample including one or more of: a type of growth media from which the clinical sample was obtained; an incubation period of time; a cell concentration in the sample; and a metabolite of interest.
14. The computer system of item 12 or 13 wherein the database is populated with reference metabolite profiles each of which was obtained by a method including:
   incubating a known cell type in a growth medium;
   after incubation, analyzing the incubated growth medium by spectrometric analysis to determine a level of each of a plurality of metabolite biomarkers in the incubated growth medium; and
   identifying a signal from the chemical analysis as the reference metabolic profile for the known cell type.
15. The computer system of item 14 wherein the reference metabolic profile is further identified according to the growth medium used for incubation and one or more other incubation parameters selected from: period of time for incubation, cell concentration in the sample, and the presence of a toxin in the growth medium.
16. A device for identifying a cell type of a cell in a sample, the device comprising:
   a. an analytical data acquisition tool configured for (i) receiving an amount of growth medium that has been incubated with the sample, (ii) conducting a chemical analysis of the growth medium to generate a metabolic profile of the growth medium, and (iii) outputting the metabolic profile; and,
   b. a computer system configured to (i) receive the metabolic profile; (ii) compare the metabolic profile with reference metabolite profiles, to identify the cell type; and (iii) generate an output of the cell type.
17. The device of item 16 wherein the analytical data acquisition tool is selected from a mass spectrometer, a nuclear magnetic resonance spectrometer or a spectrometer for optical analysis.
18. The device of any one of items 16 or 17 further comprising an incubation chamber in communication with the analytical data acquisition tool, the incubation chamber configured to contain a mixture of the sample and the growth medium and including a heater for heating the mixture.
19. The device of any one of items 16 or 17 further comprising a sample pretreatment chamber in communication with the incubation chamber.

## Claims

1. A method for identifying a cell type of a cell in a sample, comprising:
incubating the sample in a growth medium;
after incubation, analysing the incubated growth medium by chemical analysis to determine a level of each of one or more metabolites in the incubated growth medium; and
identifying the cell type of the cell by comparison of the level of each of the one or more metabolites with reference metabolite profiles and matching the level of each of the one or more metabolites with a reference metabolite profile indicative of the cell type, wherein from a panel of organisms consisting of: *Candida albicans, Candida* ssp, *Escherichia coli, Klebsiella oxytoca, Klebsiella pneumoniae, Pseudomonas aeruginosa, Enterococcus faecium, Staphylococcus aureus, Streptococcus pneumoniae,* Group A *Streptococcus, Streptococcus viridans* and coagulase negative *Streptococcus,* identifying the cell type includes:
succinate levels differentiate *E*. *coli* and *Klebsiella* ssp from all other organisms in the panel,
urocanate levels differentiate *E*. *coli* and *Klebsiella* ssp,
hydroxydecanoate differentiates *Pseudomonas aeruginosa* from all others in the panel,
arabitol levels differentiate yeast from all other organisms in the panel,
glucose levels differentiate *Pseudomonas aeruginosa* from all other Gram negative organisms in the panel;
N-acetyl-aspartate levels differentiate *Enterococcus* from all other organisms in the panel; and
xanthine differentiates *viridans streptococci* from others in the panel.
